# EUROPÄISCHE PATENTSCHRIFT

(11) **EP 3 253 338 B1**
(45) Veröffentlichungstag und Bekanntmachung des Hinweises auf die Patenterteilung: **15.04.2020**
(21) Anmeldenummer: 16703518.7
(22) Anmeldetag: 04.02.2016
(51) Int. Cl.: A61F 2/66

(54) **PROTHESENFUSS**
PROSTHETIC FOOT
PROTHÈSE DE PIED

(30) Priorität: 06.02.2015 DE 102015101746
(43) Veröffentlichungstag der Anmeldung: 13.12.2017
(73) Patentinhaber: Fraunhofer-Gesellschaft zur Förderung der angewandten Forschung e.V., 80686 München (DE)
(72) Erfinder: GROSSKOPF, Stefan, 75334 Straubenhardt (DE); STARKER, Felix, 70563 Stuttgart (DE); KRAMSKI, Andreas, 75173 Pforzheim (DE); ADLER, Andreas, 76703 Kraichtal (DE); BREUNINGER, Jannis, 72663 Großbettlingen (DE); SCHNEIDER, Urs, 70569 Stuttgart (DE)
(74) Vertreter: Mammel und Maser
(86) Internationale Anmeldenummer: PCT/EP2016/052410
(87) Internationale Veröffentlichungsnummer: WO 2016/124703

(56) Entgegenhaltungen:
- WO-A1-2007/085228
- WO-A2-2011/066354
- GB-A- 2 331 463
- GB-A- 2 387 784
- US-A- 3 335 428
- US-A1- 2010 042 228

## Beschreibung

Die Erfindung betrifft einen Prothesenfuß mit einem Gehäuse, an welchem ein Anschluss für eine Unterschenkelprothese anordenbar ist, sowie mit einem Vorfuß und einer Ferse.

Aus der US 2004/0153168 A1 ist ein Prothesenfuß bekannt, welcher eine flexible Sohle mit darauf angebrachten elastischen Elementen aufweist, um einen sogenannten Roll-over-shape zu erzielen, der dem Benutzer das Gefühl eines natürlichen Ganges geben soll. Eine solche Ausführungsform des Prothesenfußes weist den Nachteil auf, dass diese in der Herstellung aufwändig ist. Darüber hinaus ist ein solcher Prothesenfuß für einen robusten Einsatz ungeeignet. Des Weiteren ist die Anpassung eines solchen Prothesenfußes an den jeweiligen Benutzer aufwändig.

Aus der WO 2014/147070 A ist ein Prothesenfuß bekannt, welcher ein Vorfuß-Federelement, ein Fersen-Federelement und ein Sohlen-Federelement aufweist. Im Bereich eines Anschlusses an die Unterschenkelprothese sind das Vorfuß-Federelement und das Fersen-Federelement fest miteinander verschraubt. Die weiteren Verbindungen zwischen dem Fersen-Federelement und Sohlen-Federelement sowie zwischen dem Sohlen-Federelement und dem Vorfuß-Federelement erfolgen durch eine Verklebung.

Aus der US 2012/0271434 A1 ist des Weiteren ein Prothesenfuß bekannt, der mehrteilig aufgebaut ist. Ein Sohlenelement umfasst im Bereich der Ferse und den Zehen jeweils eine Tasche. Nach dem Verschrauben des zweiteiligen Fersen-Federelementes wird ein Endabschnitt des Fersen-Federelementes in die Tasche der Ferse eingesteckt. Anschließend wird ein vorderes Ende des Vorfuß-Federelementes in die vordere Tasche des Sohlen-Federelementes eingesteckt, wobei darauffolgend die jeweils einander zugeordneten Endabschnitte des Vorfuß-Federelementes und Fersen-Federelementes miteinander verschraubt werden, so dass eine feste Einspannung des Vorfuß-Federelementes und des Federelementes in den Taschen des Sohlen-Federelementes gegeben ist.

Aus der US 2010/042228 A1 ist ein Prothesenfuß mit einem Anschluss für eine Unterschenkelprothese bekannt. Dieser Prothesenfuß umfasst ein Vorfuß-Federelement, welches mit einem Sohlen-Federelement verbunden ist, sowie ein Fersen-Federelement, welches zwischen dem Sohlen-Federelement und dem Vorfuß-Federelement nahe dem Vorfuß angreift. Während einer Abrollbewegung sind das Vorfuß-Federelement und das zumindest eine Sohlen-Federelement entlang deren Längsachse gegeneinander verschiebbar.

Aus der WO 2007/085228 A1 sind ein künstlicher Fuß sowie ein Verfahren zur Steuerung seiner Bewegung bekannt. Dieser künstliche Fuß umfasst eine obere Stützstruktur sowie eine sich vom Fersenbereich in den Zehenbereich erstreckende elastische Sohlenstruktur und eine zwischen der oberen Stützstruktur und der Sohlenstruktur angeordnete elastische Verbindung. Dadurch wird ein Abrollvorgang gesteuert.

Aus der WO 2011/066354 A1 ist ein Prothesenfuß mit einem Anschluss an einer Unterschenkelprothese bekannt. Dieser Prothesenfuß umfasst ein Vorfuß-Federelement, welches fest mit einem Fersen-Federelement verbunden ist. Ein vorderes freies Ende des Vorfuß-Federelementes und ein hinteres freies Ende des Fersen-Federelementes sind in jeweiligen Aufnahmen an einem Sohlen-Federelement in Taschen eingesteckt.

Der Erfindung liegt die Aufgabe zugrunde, einen Prothesenfuß und ein Verfahren zu dessen Herstellung zu schaffen, der eine kostengünstige Herstellung ermöglicht, im Aufbau einfach ausgestaltet und robust ausgebildet ist und eine leichte Anpassung der Mechanik an den jeweiligen Benutzer ermöglicht.

Diese Aufgabe wird durch einen Prothesenfuß gelöst, bei welchem ein Gehäuse und ein Vorfuß durch zumindest ein Vorfuß-Federelement, der Vorfuß und eine Ferse durch zumindest ein Sohlen-Federelement sowie die Ferse und das Gehäuse durch zumindest ein Fersen-Federelement verbunden sind, wobei die dem Vorfuß zugeordneten Endabschnitte des zumindest einen Vorfuß-Federelementes und des zumindest einen Sohlen-Federelementes mit zumindest einer Halteeinrichtung derart zueinander positioniert sind, so dass das zumindest eine Vorfuß-Federelement und das zumindest eine Sohlen-Federelement während einer Abrollbewegung entlang deren Längsachse gegeneinander verschiebbar geführt sind. Dadurch wird ein Abrollverhalten ermöglicht, welches einem natürlichen Gehen sehr nahekommt.

Des Weiteren kann bei der erfindungsgemäßen Ausgestaltung des Prothesenfußes das Abrollverhalten durch die Federkennlinie des zumindest einen Vorfuß-Federelements bestimmt werden. Auch kann vorrangig durch das zumindest eine Sohlen-Federelement ein harmonisches Abrollen zwischen der Ferse und dem Vorfuß eingestellt werden. Ein gedämpftes Auftreten am Boden wird im Wesentlichen durch das zumindest eine Fersen-Federelement bestimmt. Durch den Einsatz solcher Federelemente für die Ausgestaltung des Prothesenfußes kann somit in einfacher Weise eine individuelle Anpassung für den jeweiligen Benutzer ermöglicht sein. Darüber hinaus sind solche Federelemente kostengünstig herzustellen und im Einsatz auch robust.

Des Weiteren sind bevorzugt die der Ferse zugeordneten Endabschnitte des zumindest einen Vorfuß-Federelementes und des zumindest einen Sohlenelementes mit zumindest einer Halteeinrichtung fest zueinander fixiert. Dadurch sind beim Auftreten im Bereich der Ferse definierte Verhältnisse zur Krafteinleitung gegeben, wohingegen mit zunehmender Abrollung durch die zumindest geringfügig gegeneinander verschiebbare Anordnung der Endabschnitte des zumindest einen Vorfuß-Federelementes und des zumindest einen Sohlenelementes im Vorfuß ein harmonisches und dynamisches Gehen erzielt wird.

Des Weiteren weist bevorzugt die Halteeinrichtung des Vorfußes und die Halteeinrichtung der Ferse zumindest einen schlitzförmigen oder U-förmigen Halteabschnitt zur Aufnahme der jeweiligen Endabschnitte des zumindest einen Vorfuß-Federelementes und des zumindest einen Sohlen-Federelementes sowie des zumindest einen Sohlen-Federelementes und des zumindest einen Fersen-Federelementes auf. Dadurch kann der Aufnahmebereich für die Endabschnitte der Federelemente quasi gleich aufgebaut sein. Zusätzlich kann dadurch in einfacher Weise eine lösbare Befestigung geschaffen sein.

Die dem Vorfuß zugeordneten Endabschnitte des zumindest einen Vorfuß-Federelementes und des zumindest einen Sohlen-Federelementes weisen vorteilhafterweise eine langlochförmige Ausnehmung oder eine U-förmige Ausnehmung auf, durch welche diese Endabschnitte durch das vorzugsweise lösbare Verbindungselement zumindest an der Halteeinrichtung des Vorfußes befestigt sind. Dadurch sind im Bereich des Vorfußes die Endabschnitte der jeweiligen einander zugeordneten Federelemente geringfügig gegeneinander verschiebbar, wodurch das Abrollverhalten des Prothesenfußes verbessert wird.

Des Weiteren umfasst bevorzugt die Halteeinrichtung des Vorfußes zumindest einen Halteabschnitt und ein Klemmelement, welche vorzugsweise lösbar zueinander sind und einen Aufnahmeraum für die Endabschnitte des zumindest einen Vorfuß-Federelementes und des zumindest einen Sohlen-Federelementes bilden. Der Halteabschnitt und der Klemmabschnitt sind vorzugsweise in einem vorbestimmten Abstand zueinander anordenbar, so dass darin die Endabschnitte des zumindest einen Vorfuß-Federelementes und das zumindest eine Sohlen-Federelement ohne Pressung zueinander angeordnet sind. Bevorzugt ist zwischen dem Halteabschnitt und dem Klemmelement eine Hülse eingeklemmt oder gepresst gehalten, so dass sich diese Hülse durch die langlochförmige Ausnehmung oder U-förmige Ausnehmung der Endabschnitte des zumindest einen Vorfuß-Federelementes oder Sohlen-Federelementes erstreckt und somit die Endabschnitte zur Hülse oder zum Aufnahmeraum im Vorfuß während dem Gehen gegeneinander verschiebbar positioniert und/oder fixiert.

Bevorzugt sind die Endabschnitte des zumindest einen Vorfuß-Federelementes und Sohlen-Federelementes im Vorfuß, die Endabschnitte des zumindest einen Sohlen-Federelementes und Fersen-Federelementes in der Ferse sowie die Endabschnitte des zumindest einen Fersen-Federelementes und Vorfuß-Federelementes an dem Gehäuse vorzugsweise lösbar befestigt. Dadurch kann für einen solchen Prothesenfuß ein quasi modularer Aufbau gegeben sein, durch welchen die zwischen dem Gehäuse, dem Vorfuß und der Ferse angeordneten Federelemente entsprechend der Anzahl und/oder der Gestaltung einsetzbar, auswählbar und/oder austauschbar sind.

Das zumindest eine Vorfuß-Federelement, das zumindest eine Sohlen-Federelement und das zumindest eine Fersen-Federelement bilden vorteilhafterweise einen dreieckförmigen Aufbau beziehungsweise positionieren den Vorfuß, die Ferse und das Gehäuse in einer dreieckförmigen Anordnung, wobei vorzugsweise ein im Wesentlichen gleichschenkliges Dreieck durch das zumindest eine Vorfuß-Federelement und das zumindest eine Sohlen-Federelement gebildet ist. Dadurch kann nicht nur ein kompakter Aufbau sowie ein optimiertes Zusammenwirken der Federelemente für die aufzunehmenden Kräfte erzielt werden, sondern insbesondere eine dem Körper nachempfundene strukturelle Bauweise geschaffen werden.

Das zumindest eine Fersen-Federelement weist bevorzugt einen U- oder V-förmigen Verlauf auf, wobei der Krümmungsbereich des U- oder V-förmigen Verlaufs in Richtung auf den Vorfuß weist. Durch diese Anordnung kann eine hohe Kraftaufnahme beim Aufsetzen der Ferse während eines Abrollvorganges des Fußes ermöglicht sein.

Die Federelemente sind bevorzugt aus einem streifenförmigen Federelement hergestellt. Bevorzugt sind die Federelemente als Blattfederelemente ausgebildet oder aus einem Polymerverbundelement. Diese können insbesondere in der Ausgestaltung als Blattfederelement beispielsweise eine Federmaterialdicke von 0,1 mm bis 3 mm aufweisen. Die Federmaterialdicke und/oder die Anzahl der Lagen der Federelemente kann in Anpassung an das Körpergewicht und/oder an die sportlichen Aktivitäten des Trägers erfolgen.

Zwischen einem am Gehäuse fest anliegenden Abschnitt des zumindest einen Vorfuß-Federelementes und dem Vorfuß an dem zumindest einen Vorfuß-Federelement ist eine Einprägung vorgesehen. Durch eine solche Einprägung, wie beispielsweise eine Sicke oder Kalotte, kann eine Vorfuß-Federkennlinie einstellbar sein. Insbesondere kann dadurch ein degressives Federverhalten eingestellt werden, wodurch beim Abrollen des Fußes kurz vor dem Abstoßen, insbesondere über einen Zehenbereich, ein erhöhter Rückfedereffekt erfolgt, der eine zusätzliche Energierückgewinnung für den Benutzer ermöglicht. Durch eine solche Einprägung kann quasi ein Schnappeffekt des zumindest einen Vorfuß-Federelementes erzielt werden, der zu einem verzögerten Rückschnappen in der Standphase des Gehens führt und somit ein dynamisches Gehen erlaubt. Gleichzeitig wird beispielsweise durch eine degressive Kennlinie des zumindest einen Vorfuß-Federelementes das Verhalten beim Gehen verbessert, da dadurch ein stabileres Stehen aufgrund der anfänglichen Steigung der Federkennlinie gegeben ist. Ergänzend oder alternativ kann zusätzlich zu der Einprägung die Anzahl der Vorfuß-Federelemente, Sohlen-Federelemente und Fersenelemente entsprechend ausgewählt und angepasst werden, wobei ergänzend und/oder alternativ die Materialauswahl als auch die Federkonstanten des jeweiligen Federelementes entsprechend angepasst und ausgewählt werden können.

Das Gehäuse des Prothesenfußes ist gemäß einer vorteilhaften Ausführungsform der Erfindung als ein Fußoberteil ausgebildet, welches einen Anschlussabschnitt für einen Anschluss an einer Unterschenkelprothese aufweist, von welchem aus sich eine Fußoberseite keilförmig in Richtung zum Vorfuß erstreckt und eine fußsohlenseitige Anlagefläche am Gehäuse vorgesehen ist, welche sich von dem distalen Ende der Fußoberseite bis zum Fersenbereich erstreckt und vorzugsweise zur Ferse ansteigend verläuft. Dieses Gehäuse bildet dadurch einerseits einen sicheren und festen Anschluss an ein Gelenk der Unterschenkelprothese und ermöglicht andererseits, dass eine sichere und definierte Anlagefläche des zumindest einen Vorfuß-Federelementes und Fersen-Federelementes gegeben ist. Gleichzeitig kann durch die Form des Fußoberteils beziehungsweise dessen Anlagefläche Einfluss auf das Abrollverhalten genommen werden, da das zumindest eine Vorfuß-Federelement zumindest zeitweise während eines Abrollvorganges sich an dieser Anlagefläche abstützt.

Vorteilhafterweise ist die Anlagefläche gekrümmt ausgebildet, insbesondere im distalen Bereich. Dabei können unterschiedliche Radien vorgesehen sein, durch welche eine Bewegungsbegrenzung und das Maß der Biegung des zumindest einen Vorfuß-Federelements eingestellt werden können.

Des Weiteren weist bevorzugt das Gehäuse distal an der Anlagefläche einen Endaufschlag auf, der eine Krümmung oder Biegung des Vorfußfederelementes bei einer Abrollbewegung begrenzt. Dadurch kann das Vorfuß-Federelement vor einer Überlast geschützt werden.

Des Weiteren ist bevorzugt an der Anlagefläche des Gehäuses eine auf das Vorfuß-Federelement gerichtete Kalotte vorgesehen. Diese Kalotte liegt vorteilhafterweise in einer Ausgangsposition des Prothesenfußes an dem Vorfuß-Federelement an. Bevorzugt kann das Vorfuß-Federelement in diesem Bereich auch eine Einprägung aufweisen, die wiederum in Richtung auf die Anlagefläche des Gehäuses hervorsteht. Durch diese Kalotte kann eine Erhöhung der Federkraft gegeben sein.

Bevorzugt sind im Fersenbereich des Gehäuses Endabschnitte des zumindest einen Vorfuß-Federelementes und des zumindest einen Fersenelementes befestigt. Dadurch ist eine möglichst lange Anlagefläche am Gehäuse gegeben, um eine Abstützung für das zumindest eine Vorfuß-Federelement zu bilden und auch ein harmonisches Abrollen zu ermöglichen, welches durch das Profil oder den Verlauf der Anlagefläche mit beeinflusst werden kann. Insbesondere ist im distalen Bereich die Anlagefläche mit einem Abrollradius versehen.

Zwischen der Ferse und dem Fersenbereich am Gehäuse ist vorteilhafterweise ein Dämpfungselement vorgesehen, welches vorzugsweise mit dem lösbaren Verbindungselement zum Gehäuse befestigt ist, welches die Endabschnitte des zumindest einen Fersen-Federelementes und Vorfuß-Federelementes fixiert. Durch die Kombination des zumindest einen Fersen-Federelementes und des Dämpfungselementes können die bei Federelementen auftretenden Schwingungen gedämpft werden, so dass ein harmonisches und dynamisches Gehen durch ein solches Fersen-Dämpfungssystem gegeben ist.

Bevorzugt ist das Dämpfungselement durch Klemmung zwischen dem Fersenbereich des Gehäuses und der Ferse fixiert. Dies ermöglicht einen einfach konstruktiven Aufbau, einen gegebenenfalls erforderlichen Austausch während der Anpassung und/oder eine vereinfachte Wartung.

Eine weitere vorteilhafte Ausgestaltung des Prothesenfußes sieht ein oder mehrere zwischen der Ferse und dem Fersenbereich des Gehäuses angeordnete Fersen-Federelemente vor, welche zwischen einer der Ferse zugeordneten hinteren Position in eine in Richtung zum Vorfuß verlagerte vordere Position veränderbar sind. Dadurch kann die Federsteifigkeit im Fersenbereich verändert werden. Beispielsweise wird diese niedriger, wenn die Fersen-Federelemente in Richtung Vorfuß verlagert werden.

Des Weiteren können bevorzugt mehrere zwischen der Ferse und dem Fersenbereich am Gehäuse angeordnete Fersen-Federelemente vorgesehen sein, welche eng aneinander liegend teilweise oder vollständig aufgefächert zueinander angeordnet sind. Letzteres heißt, dass jedes Fersen-Federelement mit Abstand zum benachbarten Fersen-Federelement angeordnet ist. Durch diese Einstellung kann die Federsteifigkeit ebenfalls verändert werden. Insbesondere im Zusammenhang mit der Positionierung dieser Fersen-Federelemente näher zum Fersenbereich oder näher zum Vorfuß kann eine sehr individuelle Anpassung des Prothesenfußes an den Träger ermöglicht sein.

Das zumindest eine Vorfuß-Federelement erstreckt sich ausgehend von einer festen Anlage an der Anlagefläche im Fersenbereich frei zum Vorfuß, wobei insbesondere während einer Abrollbewegung des Fußes die zumindest eine Vorfußfeder vollständig zur Anlage an der Anlagefläche kommt. Dadurch kann das Abrollverhalten zusätzlich zum zumindest einen Vorfuß-Federelement auch durch den Verlauf der Anlagefläche am Gehäuse bestimmt werden.

Zwischen einem fest anliegenden Abschnitt des zumindest einen Vorfuß-Federelementes und einem distalen Ende der Anlagefläche ist ein die Anlagefläche bildendes Dämpfungselement vorgesehen. Dieses Dämpfungselement kann beispielsweise als ein Dämpfungskissen ausgebildet sein. Alternativ kann auch ein vom Benutzer einstellbares Dämpfungselement vorgesehen sein, um unterschiedliche Härten der Dämpfung einzustellen. Dies kann beispielsweise ein zustellbares Dämpfungselement sein. Dieses Dämpfungselement kann des Weiteren als eine Schicht aus einem oder mehreren Elastomeren gebildet sein, welche beispielsweise an der Anlagefläche des Gehäuses anklebt, eingeklemmt oder angespritzt sein kann. Beispielsweise können elastomere Schichten oder weich-elastomere Schichten beziehungsweise Materialien vorgesehen sein. Durch das Zusammenwirken dieses Dämpfungselementes mit dem zumindest einen Vorfuß-Federelement wird ein Vorfuß-Dämpfungssystem zur Reduktion der Schwingungen für ein harmonisches und dynamisches Gehen erzielt. Ebenso kann eine Geräuschreduzierung erfolgen.

Eine weitere vorteilhafte Ausgestaltung der Erfindung sieht vor, dass das zumindest eine Vorfuß-Federelement zumindest an einem dem Dämpfungselement gegenüberliegenden Bereich die Einprägung aufweist, insbesondere eine Sicke oder Kalotte, welche in Richtung auf das Dämpfungselement ausgerichtet ist. Dies ermöglicht des Weiteren eine Bestimmung der Federkennlinie für das Abrollverhalten des Prothesenfußes.

Vorteilhafterweise erstreckt sich zumindest ein Sohlen-Federelement über den Vorfuß hinaus und bildet einen Zehenbereich. Dadurch wird der Zehenbereich integriert mit einem Sohlen-Federelement ausgebildet. Zusätzlich kann durch diese Ausgestaltung eine Erweiterung des Abrollens bis über den Zehenbereich ermöglicht sein.

Ausgehend vom vorderen Ende des Zehenbereichs, insbesondere von einer Oberseite des Zehenbereichs, erstreckt sich bevorzugt um den Zehenbereich herum und entlang einer Unterseite des Sohlen-Federelements bis zur Ferse und von dort aus bis zum Fersenbereich des Gehäuses ein Band, welches am vorderen Bereich des Zehenbereichs und im Fersenbereich fixiert ist. Dieses Band kann an einem Ende einseitig eingespannt und am anderen Ende lösbar fixiert sein. Dieses Band wirkt im Fersenbereich wie eine Achillessehne. Zusätzlich kann dadurch eine Dynamik, insbesondere eine zusätzliche Energierückgewinnung, erzielt werden.

Bevorzugt ist das Band, welches sich vom vorderen Ende des Zehenbereichs entlang des Sohlen-Federelementes und der Ferse bis zum Gehäuse erstreckt, zumindest einseitig mit einer Vorspanneinrichtung verbunden, durch welche zwischen einer Einspannstelle am Gehäuse und am Zehenbereich eine auf das Dämpfungselement wirkende Vorspannung aufbringbar ist. Das Band kann beispielsweise am Gehäuse oder am Zehenbereich fest eingespannt sein, insbesondere durch eine Klemmung, Klebung, Vernietung oder dergleichen. Am anderen Ende kann die Vorspanneinrichtung vorgesehen sein, welche in einfacher Weise betätigbar ist und das Band zwischen der Einspannstelle und der Vorspanneinrichtung auf Zug beansprucht. Dadurch kann eine Vorspannung auf das Dämpfungselement im Fersenbereich aufgebracht werden, so dass beispielsweise erst ab einer vorbestimmten Belastung, die größer als die Vorspannung des Dämpfungselementes ist, eine Dämpfung erfolgen. Dadurch ist eine Feinjustage bezüglich dem Auftreten im Fersenbereich möglich, wodurch der Tragekomfort erhöht wird.

Das zumindest eine Vorfuß-Federelement, Sohlenelement und/oder Fersenelement ist aus einem Edelstahl, einer Aluminiumlegierung, einer Stahllegierung, Verbundwerkstoff, insbesondere Eisen-Kunststoffverbundstoffe, oder aus einem Weichmetall, insbesondere Kupfer, ausgebildet, welches vorzugsweise eine Beschichtung aufweist. Ebenso könne geschmiedete Blattfedern als Federelemente eingesetzt werden. In Abhängigkeit der Einsatzorte und/oder des Gewichtes für den Benutzer können die entsprechenden Materialien ausgewählt werden. Analoges gilt für eine Beschichtung. Beispielsweise kann eine dauerhafte Beschichtung, wie beispielsweise eine Teflonbeschichtung, vorgesehen sein, wodurch auch gegenüber Feuchtigkeit oder Salzen anfälligere Materialien für das Federelement eingesetzt werden können. Alternativ können auch lösbare Beschichtungen, wie beispielsweise Fette, Wachse, korrosionshemmende Materialien oder dergleichen, vorgesehen sein, um das Federelement vor Umwelteinflüssen zu schützen. Des Weiteren können auch sogenannte Sandwich-Materialien für das zumindest eine Federelement verwendet werden, wie beispielsweise ein Carbon-Komposit, faserverstärkte Kunststoffe oder dergleichen.

Das Gehäuse und/oder der Vorfuß und/oder die Ferse bestehen bevorzugt aus einem Leichtmetall oder einem Kunststoff oder einem Verbundmaterial. Bei einem Kunststoffmaterial ist bevorzugt ungefülltes oder gefülltes Thermoplast oder Duromere oder dergleichen vorgesehen. Auch können faserverstärkte Kunststoffe eingesetzt werden. Vorteilhafterweise werden das Gehäuse und/oder der Vorfuß und/oder die Ferse als Spritzgussteil hergestellt, um die Herstellungskosten gering zu halten.

Eine vorteilhafte konstruktive Ausgestaltung sieht vor, dass der Vorfuß quer zur Sohlenlängsrichtung breiter als das Gehäuse und/oder die Ferse ausgebildet ist. Dadurch können sowohl die Standfestigkeit verbessert als auch eine erhöhte Sicherheit beim Abrollen des Prothesenfußes erzielt werden.

Der Vorfuß weist bevorzugt ein- oder beidseitig außerhalb der Aufnahme für die Endabschnitte des zumindest einen Vorfuß-Federelementes und des Sohlen-Federelementes federnd nachgiebige Stützabschnitte auf. Dadurch ist eine seitliche Stabilisierung beziehungsweise eine Querstabilisierung des Prothesenfußes ermöglicht.

Des Weiteren ist bevorzugt vorgesehen, dass das Gehäuse zumindest an der Fußoberseite eine Verrippung mit mehreren Rippen aufweist, durch deren Verlauf und/oder Größe und/oder Anzahl der Rippen die Steifigkeit des Gehäuses einstellbar ist. Dadurch kann ein weiterer Parameter gegeben sein, um eine individuelle Anpassung an den Träger der Fußorthese zu ermöglichen.

Nach einer vorteilhaften Ausgestaltung des Prothesenfußes werden das zumindest eine Vorfuß-, Sohlen- und Fersen-Federelement mit einer Schutzfolie, insbesondere einer Schrumpffolie, umhüllt und anschließend umschäumt. Dies ermöglicht eine Ausgestaltung des Prothesenfußes in Form eines natürlichen Fußes. Zusätzlich kann eine leichte Dämpfung durch die Auswahl des aufzubringenden Schaumstoffes, Polymeres oder der Vulkanisierung möglich sein.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine perspektivische Ansicht von vorne oben auf einen Prothesenfuß,
Figur 2 eine perspektivische Ansicht von oben hinten auf den Prothesenfuß gemäß Figur 1,
Figur 3 eine Ansicht von oben auf den Prothesenfuß gemäß Figur 1,
Figur 4 eine perspektivische Ansicht von vorne-unten auf den Prothesenfuß gemäß Figur 1 und
Figur 5 einen schematischen Längsschnitt des Prothesenfußes gemäß Figur 1,
Figur 6 eine schematisch vergrößerte Schnittansicht eines Vorfußes des Prothesenfußes,
Figur 7 eine perspektivische Detailansicht einer Vorspanneinrichtung für ein Band zur Dämpfungseinstellung im Fersenbereich,
Figur 8 eine schematische Schnittdarstellung entlang der Linie VII-VII in Figur 7,
Figur 9 einen schematischen Längsschnitt eines umschäumten Prothesenfußes in einem Schuh,
Figur 10 eine perspektivische Ansicht eines umschäumten Prothesenfußes mit einer Fußoptik,
Figur 11 einen schematischen Längsschnitt einer alternativen Ausführungsform eines Prothesenfußes,
Figur 12 eine perspektivische Ansicht des alternativen Prothesenfußes gemäß Figur 11,
Figur 13 einen schematischen Längsschnitt einer weiteren alternativen Ausführungsform zu Figur 11,
Figur 14 einen schematischen Längsschnitt einer weiteren alternativen Ausführungsform zu Figur 13,
Figur 15 eine schematische Ansicht von oben auf die Ausführungsform gemäß Figur 14, und
Figur 16 eine schematische Seitenansicht des Prothesenfußes gemäß Figur 14 ohne Umschäumung.

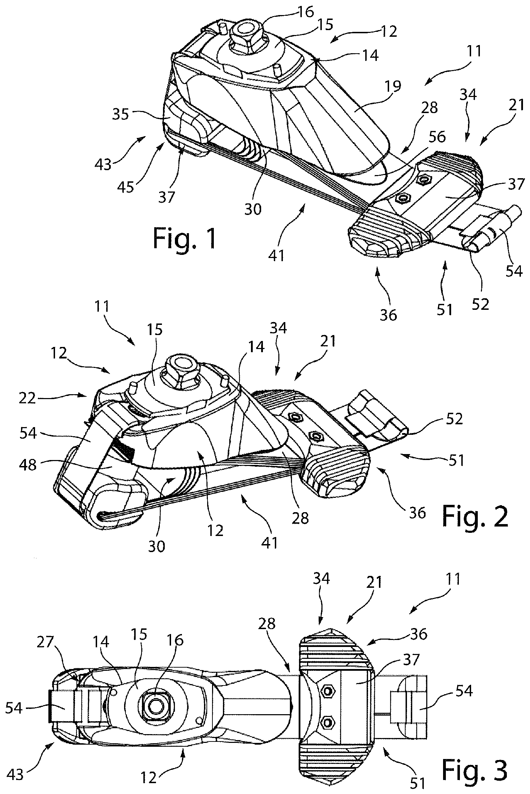

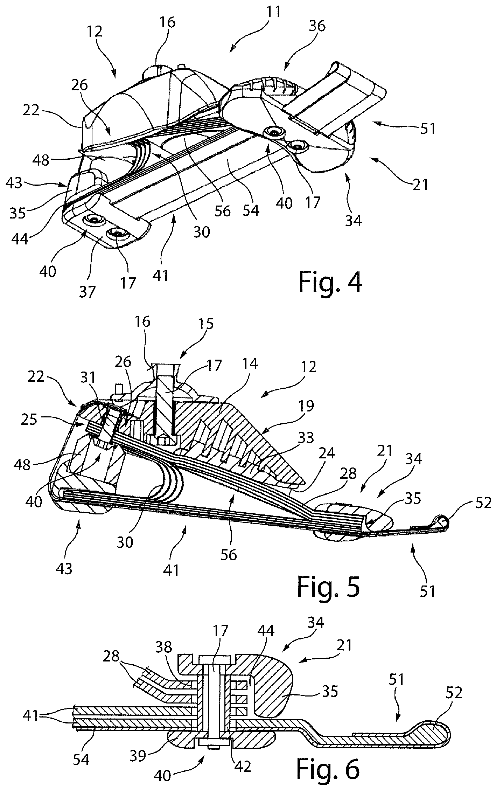

In den Figuren 1 bis 4 sind verschiedene perspektivische Ansichten eines erfindungsgemäßen Prothesenfußes 11 dargestellt. Die Figur 5 zeigt einen Vollquerschnitt in Längsrichtung des Prothesenfußes 11. Dieser Prothesenfuß 11 umfasst ein Gehäuse 12, welches an einer Oberseite einen Anschlussabschnitt 14 aufweist, an welchem verschiedene Anschlüsse 15 einsetzbar sind, um diesen Prothesenfuß 11 an verschiedene Unterschenkelprothesen, welche nicht dargestellt sind, anzubringen. Hierfür ist beispielsweise der Anschlussstutzen 16 dargestellt. Dieser Anschluss 15 ist vorteilhafterweise, wie dies in der Schnittdarstellung gemäß Figur 5 dargestellt ist, von innen durch ein Befestigungsmittel 17, insbesondere eine Schraube, austauschbar am Gehäuse 12 fixiert.

Das Gehäuse 12 bildet ein Fußoberteil und umfasst eine Fußoberseite 19, welche sich in Richtung auf einen Vorfuß 21 erstreckt. Diese Fußoberseite 19 ist einem natürlichen Verlauf eines Ristes des Fußes angepasst. Gegenüber liegend zur Fußoberseite 19 ist ein Fersenbereich 22 ausgebildet, der im Vergleich zum distalen Ende der Fußoberseite 12 in der Höhe gering ausgebildet ist. Zwischen dem Fersenbereich 22 und einem distalen Ende der Fußoberseite 19 erstreckt sich fußsohlenseitig eine Anlagefläche 24, welche mehrere Funktionsabschnitte aufweist. Im Fersenbereich 22 umfasst die Anlagefläche 24 einen Befestigungsabschnitt 26, an welchem zumindest ein Vorfuß-Federelement 28 und zumindest ein Fersen-Federelement 30 durch ein Verbindungsmittel 31 fixiert sind. Bevorzugt ist das Verbindungsmittel 31 als eine Schraubverbindung vorgesehen. Alternativ kann eine Klips-, Steck-, Rast-, Klemmverbindung oder dergleichen vorgesehen sein. Im Bereich des Befestigungsabschnitts 26 der Anlage 24 liegt das Vorfuß-Federelement 28 bevorzugt an.

An den Befestigungsabschnitt 26 der Anlagefläche 24 schließt sich in distaler Richtung ein Dämpfungselement 33, insbesondere ein Dämpfungskissen, an, an welchem das zum Dämpfungselement 33 weisende Vorfuß-Federelement 28 zumindest zeitweise beim Gehen zur Anlage kommt, wie nachfolgend noch beschrieben wird. Das Dämpfungselement 33 kann sich vorteilhafterweise bis zum distalen Ende der Fußoberseite 19 erstrecken. Das Dämpfungselement 33 kann in seiner Nachgiebigkeit vorteilhafterweise einstellbar sein.

Der Vorfuß 21 ist gegenüber dem Gehäuse 12 frei bewegbar vorgesehen, wobei die Bewegungsrichtung und der Bewegungsumfang zumindest durch das zumindest eine Vorfuß-Federelement 28 bestimmt ist. Ein dem Fersenbereich 22 gegenüberliegender Endabschnitt des zumindest einen Vorfuß-Federelementes 28 ist in einem U-förmigen oder schlitzförmigen Halteabschnitt 35 einer Halteeinrichtung 34 am Vorfuß 21 aufgenommen und fixiert. Die Halteeinrichtung 34 kann beispielsweise zweiteilig ausgebildet sein, wobei dieser benachbart zum Halteabschnitt 35 Stützabschnitte 36 umfasst sowie ein Klemmelement 39, welches zwischen den Stützabschnitten 36 eingreift, um den Halteabschnitt 35 zu bilden und die Endabschnitte des zumindest einen Vorfuß-Federelementes 28 und Sohlen-Federelementes 41 aufzunehmen. Die Stützabschnitte 36 können Längsrippen umfassen, welche sich entlang einer Fußachse erstrecken, so dass eine Querstabilität oder Erhöhung der Standfestigkeit des Prothesenfußes 11 erzielt wird.

Die Halteeinrichtung 34 fixiert sowohl das zumindest eine Vorfuß-Federelement 28 als auch das zumindest eine Sohlen-Federelement 41.

Dem Vorfuß 21 gegenüberliegend ist eine Ferse 43 vorgesehen, welche einen dem Vorfuß 21 gegenüberliegenden Endabschnitt des Sohlen-Federelementes 41 sowie ein Endabschnitt des Fersen-Federelementes 30 in einer Halteeinrichtung 45 aufnimmt. Hierzu umfasst die Halteeinrichtung 45 ebenso wie der die Halteeinrichtung 34 einen Halteabschnitt 35, in welchem die Endabschnitte des zumindest einen Sohlen-Federelementes 41 und des zumindest einen Fersen-Federelementes 30 aufgenommen sind.

Die in den Halteabschnitten 35 der Halteeinrichtung 34 angeordneten Endabschnitte des zumindest einen Vorfuß-Federelementes 28 und des zumindest einen Sohlen-Federelementes 41 weisen bevorzugt langlochförmige Ausnehmungen 38 auf, durch welche Befestigungsmittel, insbesondere lösbare Befestigungsmittel 17, wie beispielsweise Schrauben, eingreifen, um die Endabschnitte des zumindest einen Vorfuß-und Sohlen-Federelementes 28, 41 im Aufnahmeraum 44 zu fixieren, jedoch relativ zueinander verschiebbar aufnehmen. Beispielsweise kann das zumindest eine Vorfuß- und zumindest eine Sohlen-Federelement 28, 41 jeweils eine langlochförmige Ausnehmung 38 aufweisen, so dass durch den Halteabschnitt 35 das oder die Vorfuß- und Sohlen-Federelemente 28, 41 aufeinander liegend zueinander gehalten werden, jedoch entlang deren Längsachsen im Halteabschnitt 35 relativ zueinander verschiebbar sind. Beispielsweise kann auch in einem der beiden Federelemente 28, 41 eine Bohrung und in dem anderen der beiden Federelemente 28, 41 eine langlochförmige Ausnehmung vorgesehen sein, so dass die Halteeinrichtung 34 einerseits zu einem der beiden Federelemente 28, 41 fixiert und das andere der beiden Federelemente 28, 41 relativ dazu verschiebbar ist, um verbesserte Abrolleigenschaften zu erzielen. Alternativ zu den langlochförmigen Ausnehmungen kann auch eine U-förmige Ausnehmung ausgebildet sein, die stirnseitig in den Halteabschnitt 35 weisend offen ist. Der Aufnahmeraum 44 wird durch einen Halteabschnitt 35 und ein Klemmelement 39 gebildet. Dieser ist zumindest einseitig offen. Das Klemmelement 39 und der Halteabschnitt 35 sind durch eine lösbare Verbindung 40 zueinander positioniert. Bevorzugt ist die lösbare Verbindung 40 als eine Schraubverbindung ausgebildet, so dass sich beispielsweise ein Schraubbolzen durch die langlochförmigen Ausnehmungen 38 des zumindest einen Vorfuß- und Sohlen-Federelementes 28, 41 erstreckt.

Zwischen dem Halteabschnitt 35 und dem Klemmelement 39 der Halteeinrichtung 34 ist eine Hülse 42 befestigt, insbesondere zwischen dem Halteabschnitt 35 und dem Klemmelement 39 gepresst oder geklemmt gehalten, so dass der Aufnahmeraum 44 eine definierte Höhe aufweist und die Vorfuß- und Sohlen-Federelemente 28, 41 lose aufeinander aufliegen können und gegeneinander verschiebbar bleiben. Die Hülse 42 ist in den langlochförmigen Ausnehmungen 38 des Vorfuß-und Feder-Sohlenelementes 28, 41 eingesetzt. Sofern eine geringere Anzahl als die maximale Anzahl der an dem Halteabschnitt 35 befestigbaren Vorfuß-und Sohlen-Federelementen 28, 41 eingesetzt wird, kann anstelle des nicht verwendeten Federelementes 28, 41 ein Ausgleichsadapter eingesetzt werden. Dadurch kann der Abstand des fehlenden Federelementes 28, 41 überbrückt werden. Ein solcher Ausgleichsadapter kann als eine Ringscheibe oder ein Steckelement zum Fixieren an der Hülse 42 ausgebildet sein.

Eine solche bevorzugte Ausführungsform ist schematisch vergrößert in Figur 6 dargestellt.

Die vorstehenden Ausführungen und Alternativen können in Analogie für die Ferse 43 und das zumindest eine darin angeordnete Sohlen-Federelement 41 und Fersen-Federelement 30 gelten. Bevorzugt weist jedoch die Halteeinrichtung 45 der Ferse 43 einen Halteabschnitt 35 und ein Klemmelement 37 auf, durch welche ein Aufnahmeraum 44 gebildet ist, in welchem die jeweiligen Endabschnitte des zumindest einen Sohlen-Federelementes 41 und des zumindest einen Fersen-Federelementes 30 fest zueinander aufgenommen und fixiert sind.

Zwischen der Ferse 43 und dem Fersenbereich 22 am Gehäuse 12 ist ein Dämpfungselement 48, insbesondere ein Dämpfungsgummi, vorgesehen, welches durch das Verbindungsmittel 31 zum Fersenbereich 22 ebenfalls fixiert sein kann. Dieses ist gegenüberliegend an der Ferse 43, beispielsweise durch Klemmung, fixiert oder stützt sich lediglich daran ab. Durch die Auswahl - beispielsweise der Shore-Härte - kann der Grad der Dämpfung eingestellt werden.

Zumindest ein Sohlen-Federelement 41 erstreckt sich bevorzugt durch den Vorfuß 21 hindurch und bildet einen Zehenbereich 51. Bevorzugt ist am vorderen Ende des Zehenbereichs 51 eine Abrundung 52 vorgesehen. Dieser Zehenbereich 51 ist flexibel und nachgiebig gegenüber dem Vorfuß 21, so dass dieser Zehenbereich 51 ebenfalls einen Abrollbereich darstellt. Alternativ kann an dem Vorfuß 21 auch ein separater Zehenbereich 51 angeformt oder befestigt sein.

Vom Zehenbereich 51, insbesondere ausgehend von einem oberen Bereich des Zehenbereichs 51, erstreckt sich über die Abrundung 52 entlang einer Unterseite des Sohlen-Federelementes 41 bis zur Ferse 43 und durch diese hindurch bis zum Fersenbereich 22 des Gehäuses 12 ein Band 54. Dieses Band 54 ist beispielsweise am Zehenbereich 51 fixiert und am gegenüberliegenden Ende, vorzugsweise lösbar, am Befestigungsmittel 31 ebenfalls fixiert. Dieses Band 54 kann im Fersenbereich 22 auch unlösbar befestigt sein. Dieses Band 54 kann dabei derart fixiert sein, dass eine Vor- und Zugspannung zwischen den beiden Einspannpunkten des Bandes 54 wirkt, um den Abrollvorgang bezüglich seiner Harmonie oder Dynamik einzustellen.

Im dargestellten Ausführungsbeispiel sind das Vorfuß-Federelement 28, das Sohlen-Federelement 41 und das Fersen-Federelement 30 jeweils als streifenförmiges Federelement, insbesondere Blattfederelement, ausgebildet, welches beispielsweise dieselbe Streifenbreite aufweist. Alternativ können die Federelemente bezüglich der Streifenbreite alle voneinander abweichen. Gleiches gilt für das Material und/oder die Wandstärke und/oder deren Beschichtung. Zur Einstellung einer Federkennlinie beziehungsweise zur Abstimmung des Prothesenfußes 11 auf den Träger beziehungsweise Benutzer können ein oder mehrere Vorfuß-Federelemente 28, Sohlen-Federelemente 41 und/oder Fersen-Federelemente 30 vorgesehen sein. Beispielsweise können durch die Anzahl der jeweiligen Federelemente 28, 41, 30 sowohl die Federkennlinie als auch das Abrollverhalten eingestellt werden. Des Weiteren kann beispielsweise hinsichtlich der Anzahl der Vorfuß-Federelemente 28 und/oder der darin eingebrachten Einprägung der Schnappeffekt eingestellt werden, so dass beispielsweise Vorfuß-Federelemente 28 mit oder ohne Einprägung 46 miteinander kombiniert werden oder auch Vorfuß-Federelemente 28 mit verschiedenen Einprägungen miteinander kombiniert werden. Dadurch kann sich beispielsweise auch der Schnappeffekt beim Gehen für den Träger bemerkbar machen, so dass dadurch eine spürbare Dynamik erzielt werden kann. Des Weiteren können bei der Auswahl von mehreren Vorfuß-Federelementen 28, Sohlen-Federelementen 41 und/oder Fersen-Federelementen 30 auch unterschiedliche streifenförmige Federelemente ausgewählt werden, die im Material und/oder der Wandstärke und/oder der Kennlinie voneinander abweichen.

Das zumindest eine Vorfuß-Federelement 28, das zumindest eine Sohlen-Federelement 41 und/oder das zumindest eine Fersen-Federelement 30 können in einfacher Weise durch die lösbaren Verbindungen, die am Vorfuß 21 und der Ferse 43 sowie am Gehäuse 12 vorgesehen sind, ausgetauscht werden, um eine schnelle und individuelle Anpassung des Prothesenfußes an den Träger zu ermöglichen. Insbesondere ist sowohl eine Anpassung an das Gewicht als auch auf die Laufgewohnheiten des Trägers möglich. Zudem ist im Wartungsfall ein schneller Austausch gegeben.

Beim vorliegenden Ausführungsbeispiel sind die Fersen-Federelemente 30 U-förmig oder V-förmig ausgebildet, wobei deren Krümmungsbereich in Richtung auf den Vorfuß 21 weist. Alternativ können diese im Krümmungsbereich auch V-förmig ausgebildet sein. Beispielsweise sind drei Fersen-Federelemente 30 vorgesehen, welche einerseits an der Ferse 43 und andererseits zwischen dem Dämpfungselement 48 und dem Fersenbereich 22 jeweils eingespannt sind. Die Sohlen-Federelemente 41 sind vorteilhafterweise als ebene, streifenförmige Federelemente ausgebildet, welche sich geradlinig erstrecken. Diese stellen die Verbindung zwischen dem Vorfuß 21 und der Ferse 43 dar. Gleichzeitig bilden diese eine Abstützfläche für die Fersen-Federelemente 30.

Die Vorfuß-Federelemente 28 stellen eine Verbindung zwischen dem Fersenbereich 22 des Gehäuses 12 und dem Vorfuß 21 dar und können maßgeblich das Abrollverhalten bzw. den Roll-over-shape bestimmen.

Bevorzugt ist hierzu vorgesehen, dass die Vorfuß-Federelemente 28 im Bereich des Dämpfungselementes 33 eine Einprägung 56 aufweisen, durch welche die Federkennlinie des jeweiligen Vorfuß-Federelementes 28 bestimmt ist. Durch die Einbringung der Einprägung 56 wird eine nicht lineare Kennlinie für die Vorfuß-Federkennlinie erzielt, wobei diese derart eingestellt ist, dass beim Gehen ein dynamisches Abrollen ermöglicht ist, wobei insbesondere kurz vor dem Abstoßen über den Zehenbereich 51 aufgrund der Einprägung eine Art Schnappfedereffekt eintritt und somit eine Energierückgewinnung und ein verbessertes Abheben des Prothesenfußes vom Boden erzielt werden. Durch die Länge der Anlagefläche 24 und deren Krümmungsverlauf einerseits, als auch die Auswahl und die Anzahl des oder der Vorfuß-Federelemente 28 als auch der Einprägung 56 andererseits kann das Abrollverhalten bestimmt werden.

Die Länge der Fußoberseite 19 respektive die Erstreckung der Anlagefläche 24 in Richtung auf den Vorfuß 21 bestimmt eine Begrenzung einer wirkenden Überlast und kann auch entsprechend an das Gewicht des Benutzers angepasst werden.

Das zumindest eine Vorfuß-Federelement 28, das zumindest eine Fersen-Federelement 30 und das zumindest eine Sohlen-Federelement 41 stehen im Bereich deren Befestigungspunkt, also im Befestigungsabschnitt 26 am Fersenbereich 22, in den Halteabschnitten 35 des Vorfußes und der Ferse 43 miteinander in unmittelbarer Verbindung und bilden ein geschlossenes Dreieck, so dass diese miteinander wirken und sich gegenseitig beeinflussen. Dabei bestimmen im Wesentlichen das zumindest eine Vorfuß-Federelement 28 und das zumindest eine Sohlen-Federelement 41 das Abrollverhalten, wohingegen das zumindest eine Fersen-Federelement 30 im Wesentlichen das Abfedern bei Aufsetzen des Prothesenfußes 11 bestimmt.

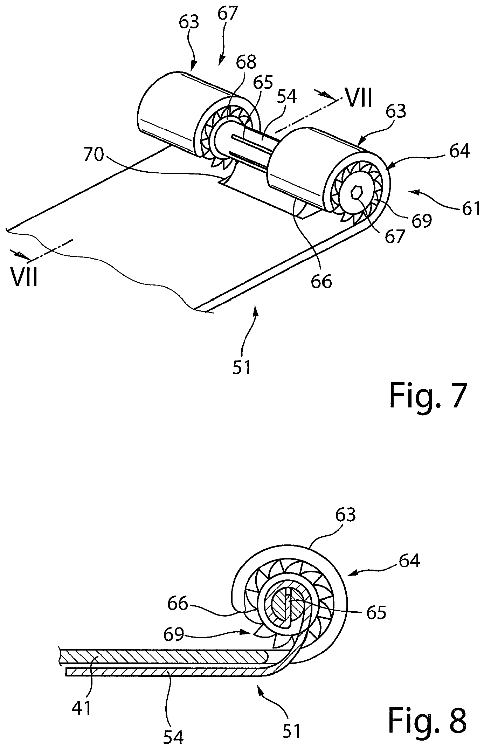

In Figur 7 ist eine perspektivische Ansicht auf einen Zehenbereich 51 dargestellt, an welchem beispielsweise eine Vorspanneinrichtung 61 für das Band 54 vorgesehen ist. Sofern die Vorspanneinrichtung 61 am Zehenbereich 51 vorgesehen ist, kann das gegenüber liegende Ende des Bandes 54 im Gehäuse 12 vorzugsweise durch die lösbare Verbindung mit dem Verbindungsmittel 31 fest zum Gehäuse 12 eingespannt sein. Alternativ kann auch in diesem Bereich eine Vorspanneinrichtung vorgesehen sein, wobei dann bevorzugt im Zehenbereich 51 das Ende des Bandes 54 durch eine Verklebung, Vernietung, Klemmung oder dergleichen fixiert ist. Alternativ kann an beiden Enden eine Vorspanneinrichtung 61 vorgesehen sein.

Die Vorspanneinrichtung 61 gemäß Figur 7 ist in einer Schnittdarstellung entlang der Linie VII-VII in Figur 8 dargestellt. Der Zehenbereich 51 weist am vorderen Ende einen gebogenen Endabschnitt 63, beispielsweise zwischen 180° und 360°, insbesondere 200° bis 300°, auf, der beispielsweise als Führung für ein Rastelement 64 dient, durch welches das Band 54 aufgewickelt werden kann. Dieses Rastelement 64 ist als eine Art Welle ausgebildet, welches im mittleren Bereich einen geringeren Durchmesser eines Wellenabschnittes 68 und einem Schlitz 65 aufweist, in welchem das freie Ende des Bandes 54 einsetzbar und sich beim Drehen des Rastelements 64 selbst verklemmt. Das Rastelement 64 weist eine sägezahnförmige Verzahnung 69 auf, welche in Spannrichtung aufgrund des freien Endabschnitts 63 drehbar ist und in entgegen gesetzter Richtung an einer Stirnkante 66 eines Endabschnitts 63 verrastend angreift, um dadurch die aufgebrachte Zugspannung auf das Band 54 aufrechtzuerhalten. Beispielsweise kann an einer Stirnseite des Rastelementes 64 eine Werkzeugaufnahme 67 vorgesehen sein, beispielsweise für einen Inbus-Schlüssel, einen Torx-Schlüssel oder einen Schraubendreher in Form eines Schlitzes oder Kreuzschlitzes. Zwischen den beiden Endabschnitten 63 ist eine Aussparung 70 vorgesehen, so dass sich das Band 54 unmittelbar auf den Wellenabschnitt 68 zwischen der sägezahnförmigen Verzahnung 69 aufwickeln lässt.

Durch diese Vorspanneinrichtung 61 kann eine Vorspannung auf das Dämpfungselement 48 im Fersenbereich 22 aufgebracht werden und dauerhaft aufrecht erhalten bleiben. Diese Vorspannung wird individuell an den Benutzer der Prothese und sein Körpergewicht angepasst.

Der vorstehend dargestellte und beschriebene Prothesenfuß 11 kann unmittelbar in dieser Ausführungsform eingesetzt werden. Bevorzugt ist jedoch vorgesehen, dass der dargestellte Prothesenfuß 11 umschäumt wird und mit einer Schutzfolie versehen ist oder nach dem Umgeben mit einer Schutz- oder Schrumpffolie eine Umschäumung erfolgt oder eine Gummimischung auf die Schutz- oder Schrumpffolie aufvulkanisiert wird, so dass die Form eines natürlichen Fußes nachgebildet ist. Der Schaumstoff kann dabei als zusätzliches Dämpfungselement dienen.

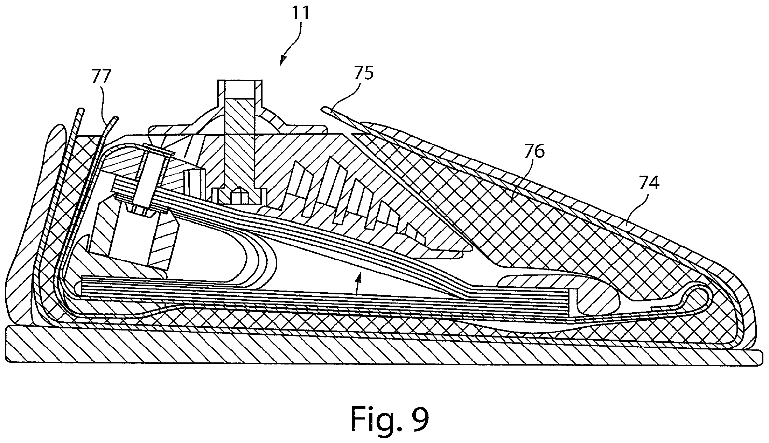

In Figur 9 ist eine schematische Schnittansicht einer weiteren alternativen Ausführungsform des Prothesenfußes 11 dargestellt. Bei dieser Ausführungsform wird der Prothesenfuß 11 umschäumt, wobei für die Formgestaltung des Fußes ein Schuh 74 des Benutzers verwendet wird, so dass die äußere Kontur des Prothesenfußes 11 eine passgenaue Form für einen Schuh 74, zumindest für eine Schuhgröße des Benutzers, aufweist.

Gemäß einer ersten Ausführungsform wird das Innere des Schuhs 74 mit einer Folie 75 ausgekleidet, welche am Einstiegsbereich des Schuhs 74 herausragt. Anschließend wird der Prothesenfuß 11 in den Schuh 74 eingesetzt. Darauf folgend wird der Innenraum des Schuhs 74 ausgeschäumt oder ausgespritzt, wodurch sich einerseits die Folie 75 an die Innenwände des Schuhs 74 anschmiegt und andererseits der Prothesenfuß 11 von dem Schaumstoff 76 oder Spritzmaterial eingehüllt wird. Bevorzugt kann vor dem Umschäumen der Prothesenfuß 11 unmittelbar mit einer Schutzfolie 77 oder Schrumpffolie umgeben sein, so dass sich der Schaumstoff 76 oder das Spritzgussmaterial außen um den Prothesenfuß 11 herum erstreckt und diesen unter Einbindung der Schutzfolie 77 oder Schrumpffolie umhüllt. Dadurch durchsetzt der Schaumstoff 76 nicht die Zwischenräume zwischen den Federelementen 28, 30, 41, so dass die einzelnen Funktionalitäten des Prothesenfußes 11 erhalten bleiben.

Nach dem Aushärten des Schaumstoffs 76 oder Spritzmaterials wird der Prothesenfuß 11 aus dem Schuh 74 herausgenommen und die Folie 75 entfernt. Der Prothesenfuß 11 ist dadurch an die spezifische Schuhgröße und/oder eine spezifische Schuhform des Benutzers angepasst.

Sofern beim Ausschäumen des Schuhs 74 bei einem eingesetzten Prothesenfuß 11 ein Integralschaum verwendet wird, genügt es als vorbereitende Maßnahme, dass die Innenwände beziehungsweise der Innenraum des Schuhs 74 mit einem Trennmittel ausgesprüht werden. Das Ausgleiten mit einer Folie 75 ist dabei nicht erforderlich.

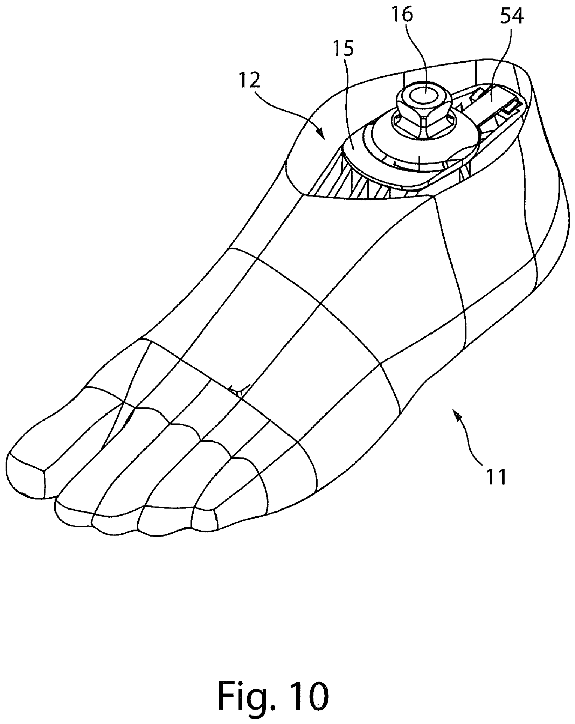

In Figur 10 ist eine perspektivische Ansicht eines umspritzten beziehungsweise umschäumten Prothesenfußes 11 dargestellt, bei welchem die Umschäumung die Form eines natürlichen Fußes aufweist. Solche Prothesenfüße 11 können an der Laufsohle eine Gummierung oder dergleichen aufweisen oder mit einem rutschfesten Material umgeben sein, so dass dieser Prothesenfuß 11 auch als Barfußprothese ausgebildet sein kann.

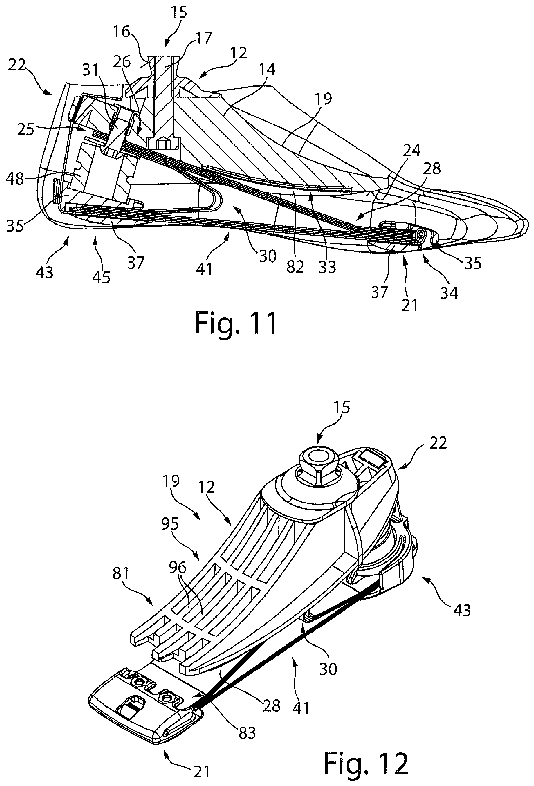

In Figur 11 ist ein schematischer Längsschnitt eines Prothesenfußes 11 gemäß Figur 10 dargestellt. Innerhalb dieses umschäumten Prothesenfußes gemäß Figur 10 kann sowohl eine der Ausführungsformen gemäß den Figuren 1 bis 9 als auch die im Schnitt in Figur 11 sowie die nachfolgend dargestellte Ausführungsform eingebracht sein.

Diese Ausführungsform gemäß Figur 11 weicht von der vorbeschriebenen Ausführungsform gemäß den Figuren 1 bis 9 dahingehend ab, dass das zumindest eine Vorfuß-Federelement 28 ohne eine Einprägung 56 ausgebildet ist. In diesem Fall werden die vorzugsweise blattfederförmig ausgebildeten Vorfuß-Federelemente einprägungsfrei eingesetzt.

Des Weiteren weicht diese Ausführungsform von der vorbeschriebenen Ausführungsform in den Figuren bis 9 dahingehend ab, dass anstelle eines als Kissen ausgebildeten Dämpfungselementes 33 auf die Anlagefläche 24 des Gehäuses 12 ein als Dämpfungsschicht ausgebildetes Dämpfungselement 33 aufgeklebt oder eingebracht wird. Dieses als Dämpfungsschicht ausgebildete Dämpfungselement 33 kann aus einem Elastomer, insbesondere Weichelastomer, ausgebildet sein. Dadurch kann wiederum eine Dämpfung und Geräuschreduzierung erzielt werden. Zudem umfasst diese Ausführungsform keinen Zehenbereich 51.

In Figur 12 ist eine perspektivische Ansicht des Prothesenfußes 11 gemäß Figur 11 vor einer Umschäumung dargestellt. Das Gehäuse 12 weist an seinem vorderen zum Verschluss 21 weisenden Ende einen Endanschlag 81 auf, durch welchen eine Biegung des Verschlusses 21 beim Abrollen in Richtung auf das Gehäuse 12 begrenzt wird. Dabei kann der Endanschlag 81 in zumindest eine Vertiefung 83, die an der Halteeinrichtung 34 am Vorfuß 21 ausgebildet ist, eingreifen. Durch einen solchen Endanschlag 81 kann das Abrollverhalten bezüglich dessen Umfang beeinflusst werden. In Abhängigkeit der Krümmung der Anlagefläche 24 und/oder der Erstreckung des Gehäuses 12 in Richtung auf den Verschluss 21 kann das Abrollverhalten beeinflusst werden.

Bei dieser Ausführungsform erstreckt sich die Anlagefläche 24 entlang der Breite des Vorfuß-Federelementes 28 und ist durch jeweils eine seitliche Schulter 82 begrenzt. Diese Schulter ermöglicht eine Führung des zumindest einen Vorfuß-Federelementes 28 zur Anlagefläche 24 während dem Abrollen, da die rechte und linke Schulter 82 jeweils an einer rechten und linken Längskante des Vorfuß-Federelementes 28 während des Abrollens vorbeigeführt wird.

Alternativ kann zumindest ein Vorfuß-Federelement 28 mit einer Einprägung 56 in Kombination mit einem als Schicht ausgebildeten Dämpfungselement 33 zum Einsatz kommen.

Bei dieser bevorzugten Ausführungsform ist vorgesehen, dass die Fußoberseite 19 des Gehäuses 12 durch eine Verrippung 95 gebildet ist. Diese Verrippung 95 umfasst mehrere Rippen 96. Hierbei können sowohl Längsrippen als auch Querrippen oder eine Kombination von Längs- und Querrippen vorgesehen sein. Durch die Breite, Größe und/oder Anzahl der Rippen 96 als auch deren Ausrichtung kann die Steifigkeit der Gehäuse 12 beeinflusst werden, das heißt, dass dieses eine größere oder geringere Elastizität aufweisen kann.

Bei dieser in den Figuren 11 und 12 dargestellten Ausführungsform ist diese Fußorthese bevorzugt ohne Zehenbereich 51 ausgebildet.

Die in den Figuren 11 und 12 dargestellte Fußorthese ist somit aufgrund des Wegfalls der Einprägung 56 und aufgrund der vereinfachten Ausgestaltung des Dämpfungselementes 33 als auch des Wegfalls des Zehenbereichs 51 vereinfacht ausgebildet und somit kostengünstig herstellbar.

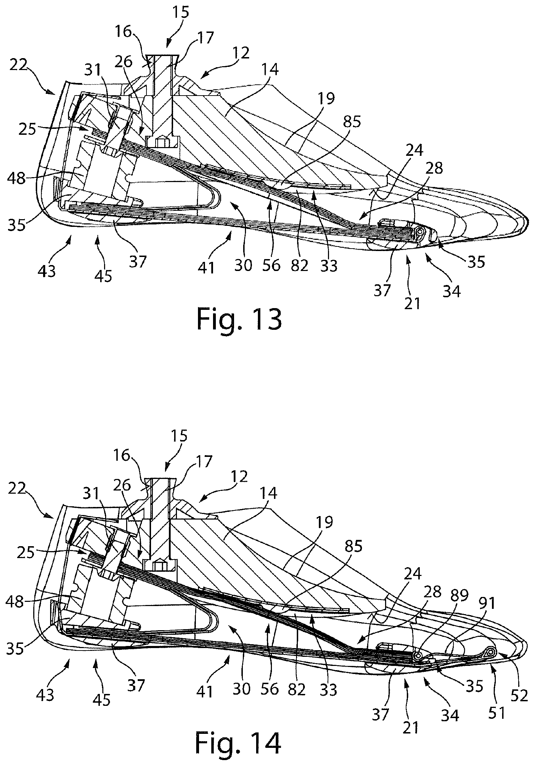

In Figur 13 ist eine alternative Ausführungsform des Prothesenfußes 11 zu den Figuren 11 und 12 dargestellt. Im Vergleich zu Figur 11 weist der Prothesenfuß 11 gemäß Figur 13 zumindest ein Vorfuß-Federelement 28 mit einer Einprägung 56 auf. Ergänzend kann an der Anlagefläche 24 des Gehäuses 12 oder einem daran angebrachten Dämpfungselement 33 eine Kalotte 85 vorgesehen sein, welche sich in Richtung auf die Einprägung 56 erhebt. Dadurch kann gezielt eine Veränderung, insbesondere eine Erhöhung der Federkennlinie, erreicht werden. Auch bei dieser Ausführungsform ist der Prothesenfuß 11 ohne den Zehenbereich 51 ausgebildet. Im Übrigen kann wiederum auf die vorbeschriebenen Ausführungsformen Bezug genommen werden.

In den Figuren 14 bis 16 ist eine weitere alternative Ausführungsform des Prothesenfußes zu Figur 13 dargestellt. Dieser Prothesenfuß 11 gemäß Figur 14 geht von der Ausführungsform gemäß Figur 13 aus. Ergänzend ist bei dieser Ausführungsform ein Zehenbereich 51 vorgesehen. Dieser Zehenbereich 51 umfasst eine alternative Ausgestaltung zu den in den Figuren 5 bis 8 dargestellten und beschriebenen Ausführungsformen. Bei dieser Ausführungsform erstreckt sich das unterste Sohlen-Federelement 41 durch die Halteeinrichtung 34 hindurch in Richtung auf die Zehen. Im vorderen Bereich ist eine Abrundung 52 vorgesehen, die durch U-förmig ausgestanzte Laschenabschnitte des Sohlen-Federelementes 41 erzielt wird. Ein mittlerer Bereich zwischen den beiden U-förmigen Schenkeln des Sohlen-Federelementes 41 ist als Laschenelement 91 ausgebildet, das in Richtung auf den Verschluss 21 zurückgebogen und zurückgeführt wird. Dies greift an einem Anschlag 89 an der Halteeinrichtung 34 an. Insbesondere umgreift das zurückgebogene Laschenelement 91 den Anschlag 89. Dadurch kann auf den Zehenbereich 51 eine Vorspannung aufgebracht werden, wodurch auch eine Winkelposition zwischen der Ausrichtung des Sohlen-Federelementes 41 zwischen dem Verschluss 21 und der Ferse 43 einerseits und dem Verschluss 21 und der Abrundung 52 des Zehenbereichs 51 andererseits eingestellt werden kann.

Die weiteren alternativen Ausführungsformen der vorbeschriebenen Prothesenfüße 11 können auch bei diesem Prothesenfuß 11vorgesehen sein.

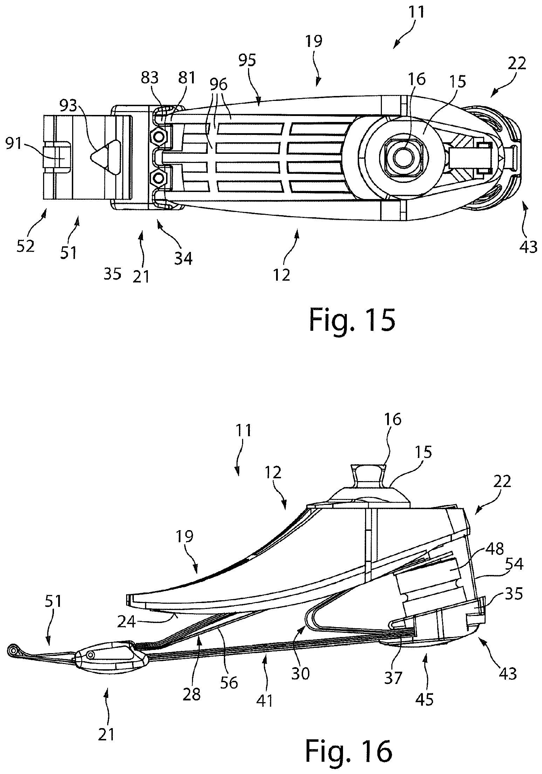

Aus der Draufsicht gemäß Figur 15 wird des Weiteren ersichtlich, dass das Sohlen-Federelement 41 in dem Zehenbereich 51 zumindest eine Aussparung 93 aufweist. Eine solche Aussparung 93 dient zur Verbesserung des elastischen Verhaltens des Zehenbereichs 51.

Des Weiteren ist aus Figur 15 zu ersehen, dass die Endanschläge 81 in U-förmigen Vertiefungen 83 an der Halteeinrichtung 34 anliegen und eingreifen können. Während eines Abrollvorganges kommt der Verschluss 21 am vorderen Bereich des Gehäuses 12 zur Anlage, wobei dann eine weitere Relativbewegung zwischen dem Verschluss 21 und dem Gehäuse 12 durch die Endanschläge 81 begrenzt werden, welche in den Vertiefungen 83 zur Anlage kommen.

## Patentansprüche

1. Prothesenfuß mit einem Gehäuse (12), an welchem ein Anschluss (15) für eine Unterschenkelprothese anordenbar ist, mit einem Vorfuß (21) und einer Ferse (43),
- bei dem das Gehäuse (12) und der Vorfuß (21) durch zumindest ein Vorfuß-Federelement (28) verbunden sind,
- bei dem der Vorfuß (21) und die Ferse (43) durch zumindest ein Sohlen-Federelement (41) verbunden sind und
- bei dem die Ferse (43) und das Gehäuse (12) durch zumindest ein Fersen-Federelement (30) verbunden sind,
- wobei die dem Vorfuß (21) zugeordneten Endabschnitte des zumindest einen Vorfuß-Federelementes (28) und des zumindest einen Sohlen-Federelementes (41) mit zumindest einer Halteeinrichtung (34) derart zueinander positioniert sind, so dass das zumindest eine Vorfuß-Federelement (28) und das zumindest eine Sohlen-Federelement (41) während einer Abrollbewegung entlang deren Längsachse gegeneinander verschiebbar geführt sind,
**dadurch gekennzeichnet,**
- **dass** zwischen einem fest am Gehäuse (12) anliegenden Abschnitt des zumindest einen Vorfuß-Federelements (28) und dem im Vorfuß (21) befestigten Endabschnitt des zumindest einen Vorfuß-Federelementes (28) am zumindest einen Vorfuß-Federelement (28) eine Einprägung (56) vorgesehen ist.

2. Prothesenfuß nach Anspruch 1, **dadurch gekennzeichnet, dass** die der Ferse (43) zugeordneten Endabschnitte des zumindest einen Sohlen-Federelementes (41) und des zumindest einen Fersen-Federelementes (30) mit zumindest einer Halteeinrichtung (45) fest zueinander in der Halteeinrichtung (45) positioniert sind.

3. Prothesenfuß nach einem der vorhergehenden Ansprüche, **dadurch gekennzeichnet, dass** die Halteeinrichtung (34) des Vorfußes (21) und die Halteeinrichtung (34) der Ferse (43) zumindest einen schlitzförmigen oder U-förmigen Halteabschnitt (35) zur Aufnahme der jeweiligen Endabschnitte des zumindest einen Vorfuß-Federelementes (28) und Sohlen-Federelementes (41) sowie des zumindest einen Sohlen-Federelementes (41) und Fersen-Federelementes (30) aufweisen.

4. Prothesenfuß nach einem der vorhergehenden Ansprüche, **dadurch gekennzeichnet, dass** die dem Vorfuß (21) zugeordneten Endabschnitte des zumindest einen Vorfuß-Federelementes (28) und des zumindest einen Sohlen-Federelementes (41) eine langlochförmige Ausnehmung (38) oder eine U-förmige Ausnehmung aufweisen, durch welche sich ein Befestigungsmittel (17) der Halteeinrichtung (34) zur Positionierung des Vorfuß-Federelementes (28) und Sohlen-Federelementes (41) in der Halteeinrichtung (34) erstreckt.

5. Prothesenfuß nach Anspruch 4, **dadurch gekennzeichnet, dass** die Halteeinrichtung (34) des Vorfußes (21) zumindest einen Halteabschnitt (35) und ein Klemmelement (39) umfasst, welche einen Aufnahmeraum (44) für die Endabschnitte des zumindest einen Vorfuß-Federelementes (28) und des zumindest einen Sohlen-Federelementes (41) bilden und der Halteabschnitt (35) und das Klemmelement (39) in einen vorbestimmten Abstand zueinander anordenbar sind und dazwischen eine Hülse (42) geklemmt gehalten ist.

6. Prothesenfuß nach Anspruch 1, **dadurch gekennzeichnet, dass** die Endabschnitte des zumindest einen Vorfuß-Federelementes (28) und des Sohlen-Federelementes (41) im Vorfuß (21), die Endabschnitte des zumindest einen Sohlen-Federelementes (41) und des Fersen-Federelementes (30) in der Ferse (43) und die Endabschnitte des zumindest einen Fersen-Federelementes (30) und des zumindest einen Vorfuß-Federelementes (28) an dem Gehäuse (12) befestigt, vorzugsweise lösbar befestigt, sind.

7. Prothesenfuß nach Anspruch 1, **dadurch gekennzeichnet, dass** das zumindest eine Vorfuß-Federelement (28), das zumindest eine Sohlen-Federelement (41) und das zumindest eine Fersen-Federelement (30) einen dreieckförmigen Aufbau bilden, wobei vorzugsweise ein im Wesentlichen gleichschenkliges Dreieck durch das zumindest eine Vorfuß-Federelement (28) und das zumindest eine Sohlen-Federelement (41) gebildet ist und insbesondere das zumindest eine Fersen-Federelement (30) einen V-förmigen Verlauf aufweist, wobei der Krümmungsbereich des V-förmigen Bereichs in Richtung auf den Vorfuß (21) weist.

8. Prothesenfuß nach einem der vorhergehenden Ansprüche, **dadurch gekennzeichnet, dass** das zumindest eine Vorfuß-Federelement (28), das zumindest eine Sohlen-Federelement (41) und/oder das zumindest eine Fersen-Federelement (30) als streifenförmiges Federelement, insbesondere Blattfederelement oder geschmiedetes Blattfederelement, ausgebildet ist, welche vorzugsweise aus Edelstahl, einer Aluminiumlegierung, einem Verbundmaterial, einer Stahllegierung oder einem Weichmetall, insbesondere Kupfer, ausgebildet ist, welches vorzugsweise eine Beschichtung aufweist, die als dauerhafte Beschichtung, insbesondere als Teflon-Beschichtung oder als lösbare Beschichtung, insbesondere aus Fett, ausgebildet ist.

9. Prothesenfuß nach einem der vorhergehenden Ansprüche, **dadurch gekennzeichnet, dass** das Gehäuse (12) als Fußoberteil ausgebildet ist, welches einen Anschlussabschnitt (14) aufweist, von welchem aus sich eine Fußoberseite (19) keilförmig in Richtung auf den Vorfuß (21) erstreckt und welches eine fußsohlenseitig angeordnete Anlagefläche (24) umfasst, die sich von dem distalen Ende der Fußoberseite (19) bis zum Fersenbereich (22) erstreckt, welche vorzugsweise in einem distalen Bereich eine Krümmung zur Bildung eines Abrollradius für das zumindest eine Vorfuß-Federelement (28) aufweist und/oder insbesondere zum Fersenbereich (22) ansteigend verläuft.

10. Prothesenfuß nach Anspruch 9, **dadurch gekennzeichnet, dass** das Gehäuse distal an der Anlagefläche (24) einen Endanschlag (81) aufweist, der eine Krümmung oder Biegung des Vorfuß-Federelementes (28) bei einer Abrollbewegung begrenzt.

11. Prothesenfuß nach Anspruch 9 oder 10, **dadurch gekennzeichnet, dass** an der Anlagefläche (24) des Gehäuses (12) eine auf das Vorfuß-Federelement (28) zugerichtete Kalotte (85) vorgesehen ist.

12. Prothesenfuß nach einem der Ansprüche 9 bis 11, **dadurch gekennzeichnet, dass** das zumindest eine Vorfuß-Federelement (28) ausgehend von einem Befestigungsabschnitt (26) an der Anlagefläche (24) im Fersenbereich (22) sich frei zum Vorfuß (21) erstreckt und vorzugsweise während einer Abrollbewegung des Prothesenfußes (11) das zumindest eine Vorfuß-Federelement (28) vollständig zur Anlage an der Anlagefläche (24) kommt.

13. Prothesenfuß nach Anspruch 12, **dadurch gekennzeichnet, dass** zwischen dem Befestigungsabschnitt (26) und einem distalen Ende der Anlagefläche (24) ein die Anlagefläche (24) bildendes Dämpfungselement (33) vorgesehen ist.

14. Prothesenfuß nach Anspruch 13, **dadurch gekennzeichnet, dass** das zumindest eine Vorfuß-Federelement (28) zumindest im Bereich des die Anlagefläche (24) bildenden Dämpfungselements (33) eine Einprägung (56), insbesondere eine Sicke oder Kalotte, aufweist, welche in Richtung auf das Dämpfungselement (33) gerichtet ist.

15. Prothesenfuß nach einem der vorhergehenden Ansprüche, **dadurch gekennzeichnet, dass** zumindest ein Sohlen-Federelement (41) sich über den Vorfuß (21) hinaus erstreckt und einen Zehenbereich (51) bildet und dass am vorderen Ende des Zehenbereichs (21) ein Band (54) angreift, welches sich entlang einer Unterseite des Sohlen-Federelementes (41) bis zur Ferse (43) und durch diese hindurch oder um diese herum bis zum Fersenbereich (22) des Gehäuses (12) erstreckt, welches am Fersenbereich (22) lösbar fixiert ist.

16. Prothesenfuß nach einem der vorhergehenden Ansprüche, **dadurch gekennzeichnet, dass** der Vorfuß (21) quer zur Sohlenlängsrichtung breiter als das Gehäuse (12) und/oder die Ferse (43) ausgebildet ist und der Vorfuß (21) ein- oder beidseitig außerhalb des Halteabschnittes (35) für die Endabschnitte des zumindest einen Vorfuß-Federelementes (28) und Sohlenelementes (41) federnd nachgiebige Stützabschnitte (36) aufweist.

17. Prothesenfuß nach einem der vorhergehenden Ansprüche, **dadurch gekennzeichnet, dass** das Gehäuse (12) zumindest an der Fußoberseite (19) einen Verrippung (95) mit mehreren Rippen (96) aufweist, durch deren Anzahl und/oder Größe und/oder Verlauf die Steifigkeit des Gehäuses (12) einstellbar ist.

18. Prothesenfuß nach einem der Ansprüche 1 bis 17, **dadurch gekennzeichnet**,
- das zumindest eine Vorfuß-Federelement (28), das zumindest eine Sohlen-Federelement (41) und das zumindest eine Fersen-Federelement (30) mit einer Schutzfolie umhüllt sind und zumindest annähernd die Form eines natürlichen Fußes durch Aufschäumen oder Aufvulkanisieren geformt ist.

## Claims

1. Prosthetic foot comprising a housing (12), on which a connector (15) for a below-knee prosthesis is arrangeable, comprising a forefoot (21) and a heel (43),
- in which the housing (12) and the forefoot (21) are connected by at least one forefoot spring element (28),
- in which the forefoot (21) and the heel (43) are connected by at least one sole spring element (41), and
- in which the heel (43) and the housing (12) are connected by at least one heel spring element (30),
- wherein in that the end portions of the at least one forefoot spring element (28) and of the at least one sole spring element (41) which are assigned to the forefoot (21) are positioned in relation to one another, by means of at least one holding device (34), in such a way that the at least one forefoot spring element (28) and the at least one sole spring element (41) are guided displaceably relative to one another along their longitudinal axis during a roll-over movement,
**characterised**
- **in that** an imprint (56) is provided on the at least one forefoot spring element, between a portion of the at least one forefoot spring element (28) lying closely against the housing (12) and the end portion of the at least one forefoot spring element (28) fastened in the forefoot (21).

2. Prosthetic foot according to claim 1, **characterised in that** the end portions of the at least one sole spring element (41) and of the at least one heel spring element (30) which are assigned to the heel (43) are positioned firmly in relation to one another by means of at least one holding device (45), in the holding device (45).

3. Prosthetic foot according to one of the preceding claims, **characterised in that** the holding device (34) of the forefoot (21) and the holding device (34) of the heel (43) have at least one slit-like or U-shaped holding portion (35) for receiving the respective end portions of the at least one forefoot spring element (28) and sole spring element (41), and of the at least one sole spring element (41) and heel spring element (30).

4. Prosthetic foot according to one of the preceding claims, **characterised in that** the end portions of the at least one forefoot spring element (28) and of the at least one sole spring element (41) which are assigned to the forefoot (21) have a slot-like cutout (38) or a U-shaped cutout, through which a fastening means (17) of the holding device (34) for positioning the forefoot spring element (28) and sole spring element (41) in the holding device (34) extends.

5. Prosthetic foot according to claim 4, **characterised in that** the holding device (34) of the forefoot (21) comprises at least one holding portion (35) and a clamping element (39), which form a receiving space (44) for the end portions of the at least one forefoot spring element (28) and the at least one sole spring element (41), and the holding portion (35) and the clamping element (39) are arrangeable at a predetermined distance from one another, and a sleeve (42) is held clamped therebetween.

6. Prosthetic foot according to claim 1, **characterised in that** the end portions of the at least one forefoot spring element (28) and of the sole spring element (21) are fastened, preferably releasably fastened, in the forefoot (21), the end portions of the at least one sole spring element (41) and of the heel spring element (30) are fastened, preferably releasably fastened, in the heel (43), and the end portions of the at least one heel spring element (30) and of the at least one forefoot spring element (28) are fastened, preferably releasably fastened, at the housing (12).

7. Prosthetic foot according to claim 1, **characterised in that** the at least one forefoot spring element (28), the at least one sole spring element (41), and the at least one heel spring element (30) form a triangular structure, wherein a fundamentally isosceles triangle is preferably formed by the at least one forefoot spring element (28) and the at least one sole spring element (41), in particular the at least one heel spring element (30) has a V-shaped extent, wherein the region of curvature of the or V-shaped region points in the direction of the forefoot (21).

8. Prosthetic foot according to one of the preceding claims, **characterised in that** the at least one forefoot spring element (28), the at least one sole spring element (41), and/or the at least one heel spring element (30) are/is formed as a strip-shaped spring element, in particular a leaf spring element or forged leaf spring element, which preferably are/is formed from high-grade steel, an aluminium alloy, a composite material, a steel alloy, or a soft metal, in particular copper, which preferably has a coating which is formed as a permanent coating, in particular as a Teflon coating or as a removable coating, in particular formed of grease.

9. Prosthetic foot according to one of the preceding claims, **characterised in that** the housing (12) is formed as a foot upper part which has a connector portion (14), from which a foot upper side (19) extends in a wedge-like manner in the direction of the forefoot (21) and which comprises a bearing face (24) arranged on the sole side, which bearing face extends from the distal end of the foot upper side (19) to the heel region (22), which preferably has a curvature, in a distal region, for forming a roll-over radius for the at least one forefoot spring element (28) and/or in particular runs in a rising manner to the heel region (22).

10. Prosthetic foot according to claim 9, **characterised in that** the housing has an end stop (81) distally on the bearing face (24), which end stop delimits a curvature or bending of the forefoot spring element (28) during a roll-over movement.

11. Prosthetic foot according to claim 9 or 10, **characterised in that** a dome (85) directed towards the forefoot spring element (28) is provided on the bearing face (24) of the housing (12).

12. Prosthetic foot according to one of claims 9 to 11, **characterised in that** the at least one forefoot spring element (28) extends freely to the forefoot (21) starting from a fastening portion (26) on the bearing face (24) in the heel region (22), and the at least one forefoot spring element (28) preferably comes completely into abutment against the bearing face (24) during a roll-over movement of the prosthetic foot (11).

13. Prosthetic foot according to claim 12, **characterised in that** a damping element (33) forming the bearing face (24) is provided between the fastening portion (26) and a distal end of the bearing face (24).

14. Prosthetic foot according to claim 13, **characterised in that** the at least one forefoot spring element (28) has the imprint (56), in particular a bead or dome, which is directed in the direction of the damping element (33), at least in the region of the damping element (33) forming the bearing face (24).

15. Prosthetic foot according to one of the preceding claims, **characterised in that** at least one sole spring element (41) extends beyond the forefoot (21) and forms a toe region (51) and **in that** a strap acts on the front end of the toe region (21) and extends along an underside of the sole spring element (41) as far as the heel (43) and through this or around this as far as the heel region (22) of the housing (12), and is releasably fixed, at the heel region (22) .

16. Prosthetic foot according to one of the preceding claims, **characterised in that** the forefoot (21), transversely to the sole longitudinal direction, is wider than the housing (12) and/or the heel (43) and the forefoot (21) has resiliently flexible supporting portions (36) on one or both sides outside the holding portion (35) for the end portions of the at least one forefoot spring element (28) and sole element (41).

17. Prosthetic foot according to one of the preceding claims, **characterised in that** the housing (12) has, at least on the foot upper side (19), a ribbed portion (95) comprising a plurality of ribs (96), wherein the rigidity of the housing (12) is adjusteable by the number and/or size and/or extent of the ribs.

18. Prosthetic foot according to any one of claims 1 to 17, **characterised in that** the at least one forefoot spring element (28), the at least one sole spring element (41), and the at least one heel spring element (30) are encased by a protective film, and then the assembly is provided at least approximately with the form of a natural foot by being covered in foam or vulcanised.

## Revendications

1. Pied prothétique pourvu d'un boîtier (12) sur lequel peut être disposé un raccord (15) pour une prothèse de membre inférieur, pourvu d'un avant-pied (21) et d'un talon (43),
- dans lequel le boîtier (12) et l'avant-pied (21) sont reliés par au moins un ressort d'avant-pied (28),
- dans lequel l'avant-pied (21) et le talon (43) sont reliés par au moins un ressort de plante de pied (41) et
- dans lequel le talon (43) et le boîtier (12) sont reliés par au moins un ressort de talon (30),
- les parties terminales dudit au moins un ressort d'avant-pied (28) et dudit au moins un ressort de plante de pied (41) qui sont attribuées à l'avant-pied (21) sont positionnées l'une par rapport à l'autre grâce à au moins un dispositif de retenue (34) de telle sorte que, pendant un mouvement de déroulement, ledit au moins un ressort d'avant-pied (28) et ledit au moins un ressort de plante de pied (41) peuvent se déplacer l'un par rapport à l'autre de manière guidée le long de leur axe longitudinal,
**caractérisé en ce que**
- une empreinte (56) est prévue sur ledit au moins un ressort d'avant-pied (28), entre une partie dudit au moins un ressort d'avant-pied (28) laquelle repose de manière fixe contre le boîtier (12) et la partie terminale dudit au moins un ressort d'avant-pied (28) laquelle est fixée dans l'avant-pied (21).

2. Pied prothétique selon la revendication 1, **caractérisé en ce que** les parties terminales dudit au moins un ressort de plante de pied (41) et dudit au moins un ressort de talon (30) qui sont attribuées au talon (43) sont positionnées, grâce à au moins un dispositif de retenue (45), de manière fixe l'une par rapport à l'autre dans ledit dispositif de retenue (45).

3. Pied prothétique selon l'une quelconque des revendications précédentes, **caractérisé en ce que** le dispositif de retenue (34) de l'avant-pied (21) et le dispositif de retenue (34) du talon (43) présentent au moins une partie de retenue (35) en forme de fente ou en forme de U en vue de recevoir les parties terminales respectives dudit au moins un ressort d'avant-pied (28) et dudit au moins un ressort de plante de pied (41) ainsi que dudit au moins un ressort de plante de pied (41) et dudit au moins un ressort de talon (30).

4. Pied prothétique selon l'une quelconque des revendications précédentes, **caractérisé en ce que** les parties terminales dudit au moins un ressort d'avant-pied (28) et dudit au moins un ressort de plante de pied (41) qui sont attribuées à l'avant-pied (21) présentent un évidement (38) en forme de trou oblong ou un évidement en forme de U à travers lequel s'étend un moyen de fixation (17) du dispositif de retenue (34) en vue de positionner le ressort d'avant-pied (28) et le ressort de plante de pied (41) dans ledit dispositif de retenue (34).

5. Pied prothétique selon la revendication 4, **caractérisé en ce que** le dispositif de retenue (34) de l'avant-pied (21) comprend au moins une partie de retenue (35) et un élément de serrage (39) qui forment un espace de réception (44) pour les parties terminales dudit au moins un ressort d'avant-pied (28) et dudit au moins un ressort de plante de pied (41) et **en ce que** la partie de retenue (35) et l'élément de serrage (39) peuvent être disposés entre eux selon un espacement prédéterminé et qu'un manchon (42) est maintenu serré entre ceux-ci.

6. Pied prothétique selon la revendication 1, **caractérisé en ce que** les parties terminales dudit au moins un ressort d'avant-pied (28) et du ressort de plante de pied (41) sont fixées, de préférence de manière amovible, dans l'avant-pied (21), que les parties terminales dudit au moins un ressort de plante de pied (41) et du ressort de talon (30) sont fixées, de préférence de manière amovible, dans le talon (43), et que les parties terminales dudit au moins un ressort de talon (30) et dudit au moins un ressort d'avant-pied (28) sont fixées, de préférence de manière amovible, au boîtier (12).

7. Pied prothétique selon la revendication 1, **caractérisé en ce que** ledit au moins un ressort d'avant-pied (28), ledit au moins un ressort de plante de pied (41) et ledit au moins un ressort de talon (30) forment une structure en triangle, de préférence un triangle essentiellement à côtés égaux étant formé par ledit au moins un ressort d'avant-pied (28) et ledit au moins un ressort de plante de pied (41), et **en ce qu'**en particulier ledit au moins un ressort de talon (30) présente un tracé en forme de V, la zone de courbure de la zone en forme de V étant tournée en direction de l'avant-pied (21).

8. Pied prothétique selon l'une quelconque des revendications précédentes, **caractérisé en ce que** ledit au moins un ressort d'avant-pied (28), ledit au moins un ressort de plante de pied (41) et/ou ledit au moins un ressort de talon (30) sont réalisés en tant que ressorts en forme de bande, en particulier en tant que ressort à lame ou ressort à lame forgé, lesquels sont réalisés de préférence en acier inoxydable, en un alliage d'aluminium, en un matériau composite, en un alliage d'acier ou en un métal doux, en particulier du cuivre, lesquels présentent de préférence un revêtement qui est réalisé en tant que revêtement durable, en particulier un revêtement en téflon, ou en tant que revêtement détachable, en particulier de la graisse.

9. Pied prothétique selon l'une quelconque des revendications précédentes, **caractérisé en ce que** le boîtier (12) est réalisé en tant que partie supérieure de pied, laquelle présente une partie de raccordement (14) à partir de laquelle une face supérieure de pied (19) s'étend en coin en direction de l'avant-pied (21), et laquelle comprend une surface de contact (24), disposée du côté de la plante de pied, laquelle s'étend depuis l'extrémité distale de la face supérieure de pied (19) jusqu'à la zone de talon (22) et laquelle présente, de préférence dans une zone distale, une courbure permettant la formation d'un rayon de déroulement pour ledit au moins un ressort d'avant-pied (28) et/ou laquelle s'étend en particulier en montant vers la zone de talon (22).

10. Pied prothétique selon la revendication 9, **caractérisé en ce que** le boîtier présente, de manière distale sur la surface de contact (24), une butée de fin de course (81) qui limite une courbure ou une flexion du ressort d'avant-pied (28) pendant un mouvement de déroulement.

11. Pied prothétique selon la revendication 9 ou 10, **caractérisé en ce qu'**une calotte (85) tournée vers le ressort d'avant-pied (28) est prévue sur la surface de contact (24) du boîtier (12).

12. Pied prothétique selon l'une quelconque des revendications 9 à 11, **caractérisé en ce que** ledit au moins un ressort d'avant-pied (28) s'étend librement vers l'avant-pied (21) à partir d'une partie de fixation (26) située sur la surface de contact (24) au niveau de la zone de talon (22), et **en ce que** de préférence pendant un mouvement de déroulement du pied prothétique (11), ledit au moins un ressort d'avant-pied (28) entre complètement en contact avec la surface de contact (24).

13. Pied prothétique selon la revendication 12, **caractérisé en ce qu'**un élément amortisseur (33) formant la surface de contact (24) est prévu entre la partie de fixation (26) et une extrémité distale de la surface de contact (24).

14. Pied prothétique selon la revendication 13, **caractérisé en ce que** ledit au moins un ressort d'avant-pied (28) présente, au moins dans la zone de l'élément amortisseur (33) formant la surface de contact (24), une empreinte (56), en particulier un collet ou une calotte, qui est tournée en direction de l'élément amortisseur (33).

15. Pied prothétique selon l'une quelconque des revendications précédentes, **caractérisé en ce qu'**au moins un ressort de plante de pied (41) s'étend au-delà de l'avant-pied (21) et forme une zone d'orteils (51) et **en ce que** sur l'extrémité avant de la zone d'orteils (21) est appliquée une bande (54) qui s'étend le long d'une face inférieure du ressort de plante de pied (41) jusqu'au talon (43) et à travers celui-ci ou autour de celui-ci jusqu'à la zone de talon (22) du boîtier (12) et qui est fixée de manière amovible à ladite zone de talon (22) .

16. Pied prothétique selon l'une quelconque des revendications précédentes, **caractérisé en ce que** l'avant-pied (21) est réalisé de manière à être, transversalement au sens longitudinal de la plante de pied, plus large que le boîtier (12) et/ou le talon (43), et **en ce que** l'avant-pied (21) présente d'un seul côté ou des deux côtés, à l'extérieur de la partie de retenue (35) prévue pour les parties terminales dudit au moins un ressort d'avant-pied (28) et dudit au moins un ressort de plante de pied (41), des parties d'appui (36) flexibles faisant ressort.

17. Pied prothétique selon l'une quelconque des revendications précédentes, **caractérisé en ce que** le boîtier (12) présente, au moins sur la face supérieure de pied (19), un nervurage (95) comportant plusieurs nervures (96) grâce au nombre et/ou à la taille et/ou au tracé desquelles il est possible de régler la rigidité du boîtier (12).

18. Pied prothétique selon l'une quelconque des revendications 1 à 17, **caractérisé en ce que** ledit au moins un ressort d'avant-pied (28), ledit au moins un ressort de plante de pied (41) et ledit au moins un ressort de talon (30) sont entourés d'un film protecteur et **en ce que** la forme d'un pied naturel est obtenue au moins de manière approximative par un procédé d'expansion ou de vulcanisation.
